# EUROPEAN PATENT SPECIFICATION

(11) **EP 4 225 153 B1**
(45) Date of publication and mention of the grant of the patent: **26.11.2025**
(21) Application number: 21877137.6
(22) Date of filing: 06.10.2021
(51) Int. Cl.: A61B 10/00, A61B 5/103, A61B 5/11, A61B 5/00

(54) **METHODS FOR DETERMINING OVULATION TIME**
VERFAHREN ZUR BESTIMMUNG DER OVULATIONSZEIT
PROCÉDÉS POUR DÉTERMINER LE MOMENT DE L'OVULATION

(30) Priority: 07.10.2020 US 202063088823 P
(43) Date of publication of application: 16.08.2023
(73) Proprietor: Medical Research & Development Fund For Health Services BNAI Zion Medical Center, 3339419 Haifa (IL)
(72) Inventor: CALDERON, Ilan, 3600700 Beth Lehem Haglilit (IL)
(74) Representative: AWA Denmark A/S
(86) International application number: PCT/IL2021/051199
(87) International publication number: WO 2022/074650

(56) References cited:
- EP-A2- 2 995 195
- WO-A1-2013/171799
- CN-A- 113 413 171
- US-A1- 2017 007 214
- US-A1- 2018 035 982
- US-A1- 2019 307 430
- US-A1- 2020 229 800

## Description

### FIELD OF THE INVENTION

Provided herein are non-invasive methods for determining or predicting ovulation time in humans, based on patterns of measured activity over a period of time.

### BACKGROUND OF THE INVENTION

The female menstrual cycle includes a series of hormonal and structural changes of the uterus and ovaries, to release an egg from the ovary and prepare and maintain the uterus lining to receive a fertilized egg. The menstrual cycle includes the ovarian cycle and the uterine cycle and is controlled by various hormones from the ovaries, pituitary gland, and the hypothalamus. The menstrual cycle normally last between 21 and 35 days in adult women, with a median length of 28 days.

The ovarian cycle includes changes that occur in the follicles of the ovary, whereas the uterine cycle includes changes in the endometrial lining of the uterus. These cycles can be divided into phases, including, follicular, ovulation, luteal, proliferative phase, and secretory phases.

In the follicular phase, follicles mature under the effect of follicle stimulating hormone (FSH), and one of the follicles will fully develop until ovulation. In ovulation, which is triggered by high levels of luteinizing hormone (LH) (LH-surge), the developed (mature) egg is released from the follicle (which is consequently ruptured) into the fallopian tubes. Unless the egg is consequently fertilized by a sperm, it will die (within about 24 hours). Ovulation occurs about midway through the menstrual cycle, after the follicular phase. The Luteal phase begins after ovulation, in which the ruptured follicle (from which the mature egg ovulated) is transformed into the corpus luteum that produces estrogen and progesterone. These hormones induce the endometrial glands to begin production of the proliferative endometrium and later into secretory endometrium, the site of embryonic growth if implantation occurs. The action of progesterone increases basal body temperature. The corpus luteum continues this paracrine action for the remainder of the menstrual cycle, maintaining the endometrium, before disintegrating into scar tissue during menses. In the absence of a fertilized egg, progesterone levels fall, and the thickened lining of the uterus is shed during the next menses, which marks the start of the next cycle.

Identifying the exact ovulation time is required in many situations: scheduling sexual relationship for the purpose of impregnation, avoiding having sexual relationship as a contraception method, and scheduling various treatments during treatment due to infertility.

Infertility in the modern age have become an issue with far-reaching implications, starting with mental stress, an impact on workplace function, an impact on the relationship between couples, and the like.

Existing non-invasive methods (such as, basal body temperature, detecting LH levels in urine samples, blood test to determine LH levels, cervical mucus viscosity, and the like) for detecting ovulation are not sufficiently accurate, and diagnosing the exact ovulation time requires performing repeated blood tests to determine related hormones levels (such as FSH and LH) and ultrasound examinations up to the ovulation time, such methods are very expensive, require cumbersome equipment and facilities, and causes patient distress and time waste for the patient.

International Patent Application Publication No. WO 2013171799 is directed to biorhythm-estimating device for detecting menstruation. International Patent Application Publication No. WO 2021037724 is directed to System and method for determining time interval of fertility. US 2019/307430 A1 is directed to non-invasive monitoring of estrogen of a female human. US 2017/007214 A1 is directed to unobtrusive fertility tracking, comprising a sensor for obtaining a heart signal, a processing unit configured to determine a subject's heart rate from the heart signal, and an evaluation unit configured to analyse the subject's heart rate to predict likelihood of ovulation. US 2020/229800 A1 is directed to system and method and corresponding computer program for unobtrusively determining a fertile window using sensors embedded in a bra, based on respiration parameters. CN 113 413 171 A is directed to a method for predicting ovulation day by collecting data through wearable equipment, wherein the data includes obtaining body temperature data. WO 2013/171799 A1 is directed to a biorhythm estimation device, based on measuring/calculating the respiratory frequency.

Nevertheless, there is a need in the art for improved, accurate, cost effective and time-efficient, non-invasive methods to determine and predict ovulation stage/occurrence in human female subjects.

### SUMMARY OF THE INVENTION

The invention is defined by the independent claims. Advantageous embodiments appear from the dependent claims. According to some embodiments, there are provided advantageous methods and devices for determining or predicting ovulation time in human subject by non-invasive means, based on patterns of physical activity of the subject over a period of time. In some embodiments, the patterns include determined or calculated changes (or trends thereof) in activity (such as, for example, movement, walking, running, the number of steps and/or rate of steps), over a time period, which may include, for example, the entire period or portions of the menstrual cycle. In some embodiments, the patterns of activity may be determined during at least part of the waking hours, when the subject is awake and optionally active/functioning (i.e., moving, standing, walking, running, jogging, exercising, changing position, etc.)

Without being bound to any theory or mechanism, the present invention is based at least in part on the surprising finding that in fertile human subjects, patterns of physical activity (including, for example, changes in the daily number and/or rate of steps over a period of time), are indicative of ovulation. The present invention is further based, in part, on the finding that the hormonal changes (fluctuations during menstrual cycle), affect at least some of the woman's daily functioning/activity, in particular, before and around ovulation time. In some embodiments, by identifying such patterns (including for example, changes and/or trend of changes in measured activity), accurate prediction or determination of ovulation is facilitated by non-invasive means. In some embodiments, one or more additional physiological parameters may be determined in addition to physical activity/function (such as, for example, daily number and/or rate of steps, and data related to such parameters) may be used in calculations to determine or predict ovulation.

In some embodiments, advantageously, the use of data related to one or more additional (non-invasive) parameters (such as, sleep pattern (for example, length and/or quality of sleep), heart rate, ECG, temperature, and the like), together with determination of pattern in measured activity (including, for example, changes in number and/or rate of steps and data related to such parameters) allows synergistic results, to further increase the reliability and/or accuracy of ovulation determination or prediction.

Thus, the advantageous non-invasive method and device disclosed herein can be utilized to accurately diagnose, determine and/or predict the time of ovulation, thereby aiding women subjects to schedule sex in order to conceive or avoid sex as contraceptive, as well as schedule fertility treatments as part of fertility treatment regime for IVF procedures.

According to some embodiments, there is provided a non-invasive method for determining or predicting ovulation timing (occurrence) in human subject, the method comprising:
measuring activity of the subject, over a period of time, said activity comprising steps taken by the subject; and
determining a pattern in the steps of the subject, over the period of time; wherein the pattern is indicative of ovulation.

According to some embodiments, the measured activity of the subject may further include body movement, amount of movement, intensity of movement, rate of movement, velocity of movement, repetition of movement, time length of movement, posture changes, or any combination thereof.

According to some embodiments, the measured steps may include: total number of steps, daily number of steps, number of steps per time unit (for example, minutes, hours), average number of steps, rate of steps, average rate of steps, daily rate of steps, length of steps, average length of steps, or any combination thereof. Each possibility is a separate embodiment.

According to some embodiments, the activity of the subject is measured during waking hours of the subject.

According to some embodiments, the pattern may include one or more changes in the measured activity values during one or more interim periods of the period of time, and/or one or more changes in parameters derived from the measured activity values during one or more interim periods of the period of time.

According to some embodiments, the parameters derived from the measured changes may include, for example, normalized values of the parameters, intraday day values of the parameters, trends of the values, or any combination thereof.

According to some embodiments, an increase in the measured activity value in an interim time period may be indicative of ovulation time.

According to some embodiments, an increase in parameters derived from the measured activity values may be indicative of ovulation time.

According to some embodiments, an increase in an increase in the daily number of steps, daily rate of steps, average number of daily steps, average number of steps and/or rate of steps, and/or in parameters derived therefrom, in an interim time period, may be indicative of ovulation time.

According to some embodiments, the method may further include measuring one or more physiological parameters of the subject and determining a pattern in the physiological parameters over the period of time.

According to some embodiments, the physiological parameters may be selected from: sleep patterns (such as, length of sleep, stages of sleep, quality of sleep, etc.), heart rate, heart rate variability, ECG, stress, body and/or skin temperature, oxygen saturation, SpO2, respiration rate, or any combination thereof. Each possibility is a separate embodiment.

According to some embodiments, the pattern of the one or more physiological parameters may include one or more changes in the measured physiological parameters during one or more interim periods of the period of time, and/or one or more changes in parameters derived from the changes in the measured physiological parameters during one or more interim periods of the period of time.

According to some embodiments, the time period may be in the length of about 2-45 days.

According to some embodiments, the time period may be in the length of about 14-28 days.

According to some embodiments, the interim period may be equal to or shorter than the time period.

According to some embodiments, the activity measurements may be performed continuously over the period of time. According to some embodiments, the activity measurements may be performed intermittently over the period of time.

According to some embodiments, one or more of the physiological parameter measurements may be performed continuously over the period of time. According to some embodiments, one or more of the physiological parameter measurements may be performed intermittently over the period of time.

According to some embodiments, the pattern may be determined over the entire time period, or over and between portions of the time period (interim time periods).

According to some embodiments, at least one of the calculations may be performed using a processing unit.

According to some embodiments, one or more of the measurements may be performed using a wearable device.

In some embodiments, the wearable device my include one or more suitable sensors, including, for example, but not limited to: acceleration sensor, gyroscope, movement sensor, pressure sensor, heart rate sensor, temperature sensor, light sensor, Oxygen sensor, SpO2 sensor, stress sensor, and the like, or any combinations thereof.

According to some embodiments, the processing unit may be comprised within the wearable device. According to some embodiments, the processing unit may be external to the wearable device.

According to some embodiments, data obtained by the wearable device may be processed by the processing unit, and wherein the wearable device and the processing unit are configured to operate in conjunction.

According to some embodiments, the method may further include alerting the subject when ovulation time is occurring or is expected. In some embodiments, the alert may include tactile alert, audible alert and/or visual alert.

According to some embodiments, there is provided a wearable device for determining or predicting ovulation time in a human subject, the device includes:
a sensor configured to measure activity of a subject wearing the device, over a period of time, said activity comprising steps taken by the subject; and
a processing unit configured to determine a pattern of the measured steps wherein the pattern is indicative of ovulation.

According to some embodiments, the activity of the subject measured by the device may further include body movement, amount of movement, intensity of movement, length of movement, posture changes, or any combination thereof.

According to some embodiments, the steps measured by the device may include: total number of steps, daily number of steps, number of steps per time unit, average number of steps, rate of steps, average rate of steps, daily rate of steps, length of steps, average length of steps, or any combination thereof.

According to some embodiments, the pattern determined by the processing unit of the device may include one or more changes in the measured activity values during one or more interim periods of the period of time, and/or one or more changes in parameters derived from the measured activity values during one or more interim periods of the period of time. According to some embodiments, parameters derived from the measured values may include, for example, normalized values of said parameters, intraday day values of the parameters, trends of said values, or any combination thereof.

According to some embodiments, an increase in the activity measured by the device in an interim time period may be indicative of ovulation time. According to some embodiments, an increase in parameters derived from the measured values as determined by the processing unit may be indicative of ovulation time. According to some embodiments, an increase in the daily number of steps, daily rate of steps, number of steps per time unit, average number of daily steps, average number of steps and/or rate of steps, and/or in parameters derived therefrom, as determined by the processing unit may be indicative of ovulation time.

According to some embodiments, the device may include one or more sensors for measuring one or more physiological parameters of the subject. According to some embodiments, the physiological parameters may be selected from: sleep quality, ECG, stress, temperature, oxygen saturation, heart rate, respiration, or any combination thereof.

According to some embodiments, the sensors may be selected from: stress sensor, accelerometer, gyroscope, heart rate sensor, pressure sensor, respiration sensor, temperature sensor, SpO2 sensor, or any combination thereof.

According to some embodiments, the pattern of the one or more physiological parameters may include one or more changes in the measured physiological parameters during one or more interim periods of the period of time, and/or one or more changes in parameters derived from the changes in the measured physiological parameters during one or more interim periods of the period of time.

According to some embodiments, the pattern of the one or more physiological parameters as determined by the processor, may include one or more changes in the measured physiological parameters during one or more interim periods of the period of time, and/or one or more changes in parameters derived from the changes in the measured physiological parameters during one or more interim periods of the period of time.

According to some embodiments, the device may measure the activity continuously over the period of time. According to some embodiments, the device may measure the activity intermittently over the period of time.

According to some embodiments, the device may measure one or more of the physiological parameters continuously or intermittently over the period of time or performed.

According to some embodiments, the pattern may be determined over the entire time period, or over and between portions of the time period.

According to some embodiments, the processing unit may be comprised within the wearable device. According to some embodiments, the processing unit may be external to the wearable device.

According to some embodiments, the device may further include an alert unit configured to issue indication regarding ovulation occurrence. According to some embodiments, the indication may be selected from tactile alert, audible alert and/or visual alert. According to some embodiments, the alert may be issued at a predetermined time period prior to ovulation, or at ovulation time.

Further embodiments, features, advantages and the full scope of applicability of the present invention will become apparent from the detailed description and drawings given hereinafter. However, it should be understood that the detailed description, while indicating preferred embodiments of the invention, are given by way of illustration only, since various changes and modifications will become apparent to those skilled in the art from this detailed description.

### BRIEF DESCRIPTION OF THE FIGURES

**Fig. 1** - a block diagram of steps in a method for determining or predicting ovulation based on activity, according to some embodiments; and
**Fig. 2** - a block diagram of exemplary method for determining ovulation, based on changes in daily number and/or rate of steps, according to some embodiments.

### DETAILED DESCRIPTION OF THE INVENTION

The principles, uses, and implementations of the teachings herein may be better understood with reference to the accompanying description and figures. Upon perusal of the description and figures present herein, one skilled in the art will be able to implement the teachings herein without undue effort or experimentation. In the figures, same reference numerals refer to same parts throughout.

To facilitate an understanding of the present invention, a number of terms and phrases are defined below. It is to be understood that these terms and phrases are for the purpose of description and not of limitation, such that the terminology or phraseology of the present specification is to be interpreted by the skilled artisan in light of the teachings and guidance presented herein, in combination with the knowledge of one of ordinary skill in the art.

As referred to herein, the term "activity" relates to any type of movement or physical activity a female subject is performing and that is capable of being measured/determined over a period of time. In some embodiments, activity may include, for example, body movement, including, upper body movement and/or lower body movement, rate of movement, velocity of movement, repetition of movement, gait movement, changes in posture (for example, from laying down to standing, from sitting to standing, etc.), walking, running, jogging, exercising, and the like. In some embodiments, at least part of the activity may be measured as steps taken by the subject. In some embodiments, activity is measured when the subject is awake.

According to some embodiments, provided herein are advantageous non-invasive method and device for determining or predicting ovulation time/event/phase in fertile human subject, based at least on determined or calculated pattern of measured activity (such as, for example, changes (or trends thereof) in the number of measured steps and/or rate of steps), over a time period.

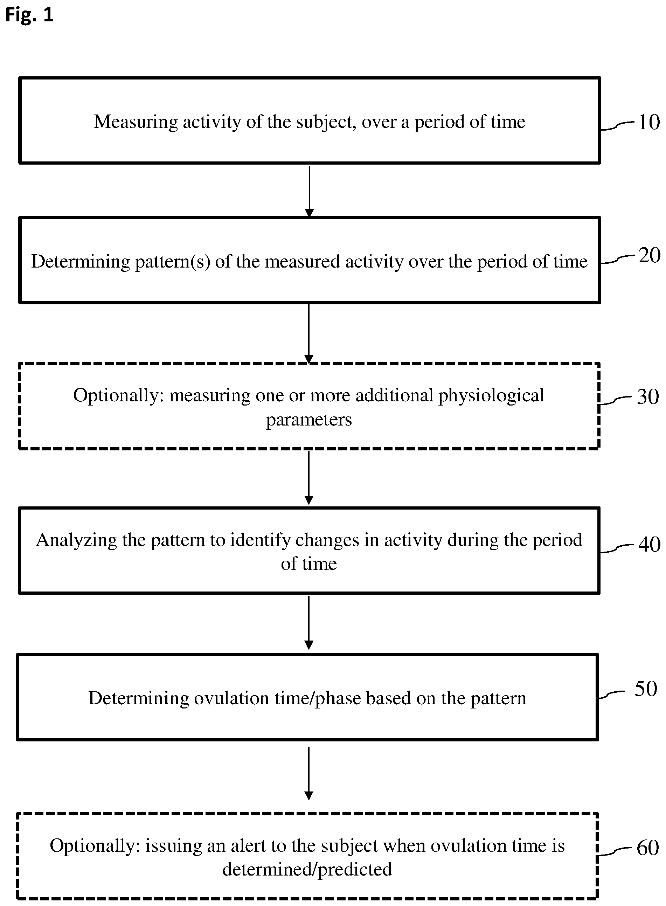

Reference is now made to **Fig. 1****,** which shows a block diagram of a method for determining ovulation, according to some embodiments. As shown in Fig. 1, at step 10, activity of the subject over a period of time is measured, using one or more suitable sensors, harbored in a corresponding device, such as, for example, a wearable device. Next, at step 20, patterns in the measured activity are determined. Next, at optional step 30, one or more additional parameters may be measured (using suitable sensors), including, for example, but not limited to: heart rate, sleep pattern, ECG, temperature, and the like. At step 40, the determined pattern(s) are analyzed to identify changes in measured activity (values as is, or parameter values derived therefrom, as detailed below), and optionally also in the measured parameters, during interim time portions of the time period or over the entire time period. In some embodiments, the determination of the pattern(s) may include any suitable calculations and statistical methods, including, for example, smoothing, averaging, normalizing, and the like. In some embodiments, the determined patterns may indicate changes in one or more measured or calculated parameters (i.e., parameters derived from the measured values), over the entire time period or over portions thereof. For example, in some embodiments, the pattern may indicate a change in activity over and between portions of the time period (i.e., interim time points). In some embodiments, the pattern may indicate changes over and between the follicular stage, ovulation stage and/or post ovulation stage (luteal stage). At step 50, the ovulation timing is determined/calculated/computed based on the determined pattern (based, for example, on identifying changes in the measured values or parameters derived/related thereto (such as, normalized value, trends thereof, and the like), during the time period). In some embodiments, increase in the activity over the time period, or at least over portions of the time period is indicative of ovulation and can be used to determine or predict ovulation time/stage/phase/period. Optionally, at step 60, the method may further include a step of issuing an alert (such as, tactile alert, audible alert, visual alert, text message, email, and the like) to the user if/when ovulation time/phase is determined or is expected/predicted to occur.

In some embodiments, the methods disclosed herein are at least partially computerized method. In some embodiments, the at least some of the steps in the methods disclosed herein are executed by one or more suitable processing units.

According to some embodiments, for the calculation/computing (processing steps) for determining or predicting ovulation time, various activity parameters (values) or data related to or derived from the values may be used. For example, such activity parameter values or data related thereto or derived therefrom may include, for example, but not limited to: length of activity, type of activity, rate of activity, intensity of activity, number of posture changes, number of steps, average number of steps (per time unit or period of time, such as, for example, average daily number of step), rate of steps, average rate of steps (per time unit or period of time), various statistical deviations and/or variances, trends in changes of the measurements or data related thereto, and the like, or any combination thereof. Each possibility is a separate embodiment.

According to some embodiments, for the calculation/computing (processing steps) for determining or predicting ovulation time, values of the activity parameters may be used or is, or may be processed by various statistical tools, including, for example, by normalization relative to a baseline, to reduce variability of the measured activity values over time intervals, by reducing background readings (background noise). In some embodiments, the baseline may be a personalized baseline (i.e., of the specific subject) or any other designated baseline (for example, of a population of subjects). In some embodiments, baseline values may be determined by providing/collecting baseline measurements of the subject over selected periods of time (such as, for example, in the range of hours, days, weeks, months, etc.). In some embodiments, the normalization of the values may improve accuracy of the results and reduce enhanced variability that may be present over short or long time intervals. In some embodiments, the normalization standardizes data representation (for example, per day and per patient) and facilitates robust thresholding. In some embodiments, normalization is used to change the values in a controlled fashion in order to standardize them. For example, in some embodiments activity per time length (for example, an hour) and/or activity per posture and/or activity for entire awake time, and the like, can be used in determining/setting robust threshold, for determining ovulation onset/time.

In some embodiments, measured activity (such as, for example number of steps) may be determined over a time period to identify/determine patterns in the activity, wherein the pattern may include, for example, changes in the measured activity values or in values related thereto over one or more time periods, including, for example, but not limited to: number of steps, daily number of steps, average number of steps, rate of steps, daily rate of steps, average rate of steps, cadence, average cadence, length of steps, average length of steps, normalized number of steps, normalized daily number of steps, normalized average number of steps, normalized rate of steps, normalized daily rate of steps, normalized average rate of steps, normalized cadence, normalized average cadence, normalized length of steps, normalized average length of steps, and the like, any statistically related value thereof, any or any combination thereof. Each possibility is a separate embodiment.

According to some embodiments, the measured steps may include: total number of steps, daily number of steps, number of steps per time unit (for example, minutes, hours), average number of steps, rate of steps, average rate of steps, daily rate of steps, length of steps, average length of steps, or any combination thereof. In some embodiments, intraday variance of the values may be used for determining daily values (such as, daily number of steps, daily rate of steps, etc.). In some embodiments, weighted averages of the values may be used for determining parameters values (such as, daily number of steps, daily rate of steps, etc.). Each possibility is a separate embodiment.

In some embodiments, to determine number of steps (such as average number of steps, rate of steps, etc.), per a time period, such as, for example, a daily number of steps, measurements performed during interim time points over the day may be used in order to determine the daily number of steps. Such interim time points may be in the length of about 1-120 minutes, about 1-24 hours, or any subranges therebetween. In some embodiments, such interim time points may include various times of day, for example, dawn hours, morning hours, noon-hours, afternoon hours, evening hours, night hours, and the like, or any combination thereof. Each possibility is a separate embodiment.

According to some embodiments, the pattern may include one or more changes in the measured activity values during one or more interim periods of the period of time, and/or one or more changes in parameters derived from the measured activity values during one or more interim periods of the period of time. In some embodiments, interim time periods may be in the range of minutes, hours, days and/or weeks. In some embodiments, interim time periods may be in the range of, for example, about 1-120 minutes, about 1-24 hours, about 1-7 days, about 1-12 weeks, or any subranges thereof.

According to some embodiments, for the calculation/computing (processing steps) for determining or predicting ovulation time, various additional physiological parameters may be used, and various values or data related to or derived from the parameters may be used. Such physiological parameters may include, for example, heart rate, heart rate variability, respiration, respiration rate, sleep pattern (for example, length of sleep, quality of sleep, sleep stages, etc.), electrocardiograms (ECG), body temperature, skin temperature, oxygen saturation, and the like. The calculations or data related to such parameters may include, for example, various mathematical and statistical analyses (such as averaging, normalization, variances, smoothing, noise/background/artifact reduction), statistical deviations, trends in changes of the measured parameters or data related thereto, and the like, or any combination thereof. Each possibility is a separate embodiment.

In some embodiments, for the calculation steps, one or more of the activity parameters and/or data related thereto may be used (as is, or after processing, as detailed above). In some embodiments, if some activity parameters or data related to or derived therefrom deviate from a predetermined threshold, they may be disregarded in the calculations.

In some embodiments, for the calculation steps, one or more personalized data related to a specific subject may be used, including, for example, medical history of the subject, relevant demographic data, baseline values of activity, baseline values of one or more physiological parameters, and the like, or any combination thereof.

In some embodiments, for the calculation steps, one or more of the physiological parameters and/or data related thereto may be used, in addition to the activity parameters. In some embodiments, if some physiological parameters or data related to or derived therefrom deviate from a predetermined threshold, they may be disregarded in the calculations.

According to some embodiments, the measurements of the activity parameters and/or the physiological parameters may be performed continuously, or essentially continuously over a period of time. In some embodiments, the measurements may be performed intermittently, over a period of time. In some embodiments, some of the measurements may be performed continuously (or essentially continuously) and other measurements maybe preformed intermutually. In some embodiments, at least some of the measurements may overlap. In some embodiments, at least some of the measurements are performed daily. In some embodiments, at least some of the calculations may be performed at least once a day. In some embodiments, at least some of the measurements are performed intraday (i.e., during different times/hours of the day for any desired time lengths).

According to some embodiments, a change in measured parameters (values or data related/derived therefrom) may be a relative change. For example, a change may be determined in percentages. In some embodiments, a trend in a change of measured parameters (values or data related thereto) may be determined and used in the calculations. In some embodiments, a change in a measured parameter (value, data related to or derived therefrom (for example, trend)) may be an increase or decrease over a period of time. In some embodiments, a change in a measured parameter (value, data related to or derived therefrom), may be of at least 1%, at least 5%, at least 10%, at least 20%, at least 30%, at least 40%, at least 50%, at least 60%, at least 70%, at least 80%, at least 90%, at least 95%, at least 99%. In some embodiments, a change in a measured parameter (value, data related to or derived therefrom), may be in the range of about 1-99%, at least 2-90%, at least 5-80%, at least 10-60%, at least 20-50%, or any subranges thereof.

According to some embodiments, the time period over which the measurements may be performed may be any suitable time range. In some embodiments, the time period may correlate with the menstrual cycle of the human subject, or stages/phases thereof (such as, follicular development stage/period; ovulation stage/period; post ovulation stage). In some embodiments, the time period is personalized, based, for example, on the physiological parameters, medical parameters and/or personal parameters the subject. In some embodiments, the time period may be in the range of about 2-180 days, or any subranges thereof. In some embodiments, the time period may be in the range of about 2-90 days, or any subranges thereof. In some embodiments, the time period may be in the range of about 2-45 days, or any subranges thereof. In some embodiments, the time period may be in the range of about 7-35 days, or any subranges thereof. In some exemplary embodiments, the time period may be in the range of about 14-28 days. In some embodiments, the time period may start on the second day of the female period until three days after ovulation.

In some embodiments, the patterns of the obtained measurements may include, for example, changes over time of the measurements. In some embodiments, changes between the obtained measurements (or data related thereto or derived therefrom) may be compared therebetween over the entire measurement period and/or between interim time points, such as, for example, time points corresponding to various stages of the menstrual cycle (such as, follicular stage, ovulation and post ovulation stage). In some embodiments, the changes in the parameters or data related thereto or derived therefrom may be determined between various stages of the menstrual cycle. For example, the measurements may be determined during the follicular stage, during LH-surge stage (ovulation) and after ovulation.

According to some embodiments, an increase in activity (as determined based, for example, on increase in number of steps, determined as described herein) is correlated with increase in LH levels. In some embodiments, an LH-surge may be correlated with a peak in the measured activity, rendering such peak in activity an indicator or predictor of ovulation time.

According to some embodiments, changes in measured activity (for example, changes in normalized average number of steps), are indicative/predictive of ovulation time in human female subjects.

According to some embodiments, the calculations may be performed using a processing element or processing unit. In some embodiments, the processing unit may be included/housed in the wearable device and the calculations are performed therewith. In some embodiments, the processing unit may be included is a separate computing device, and is configured to perform calculations on data received from the wearable device. In some embodiments, the wearable device and the processing unit are physically and/or functionally connected. In some embodiments the wearable device and the processing unit can wirelessly or physically relay data therebetween.

In some embodiments, the wearable device may include a smart watch, a smart phone, a step counter, and the like. In some embodiments, the method may be executed as an application, such as, a mobile application. In some embodiments the device may include one or more of: suitable sensor(s) (such as, step counter, thermometer, pulse sensor, movement sensor, accelerometer, gyroscope, pulse oximeter, SpO2 sensor, stress sensor, pressure sensor, light sensor, and the like, or any combination thereof), processing unit, memory, display, speaker, a communication unit, power source, a user interface, and the like, or any combination thereof.

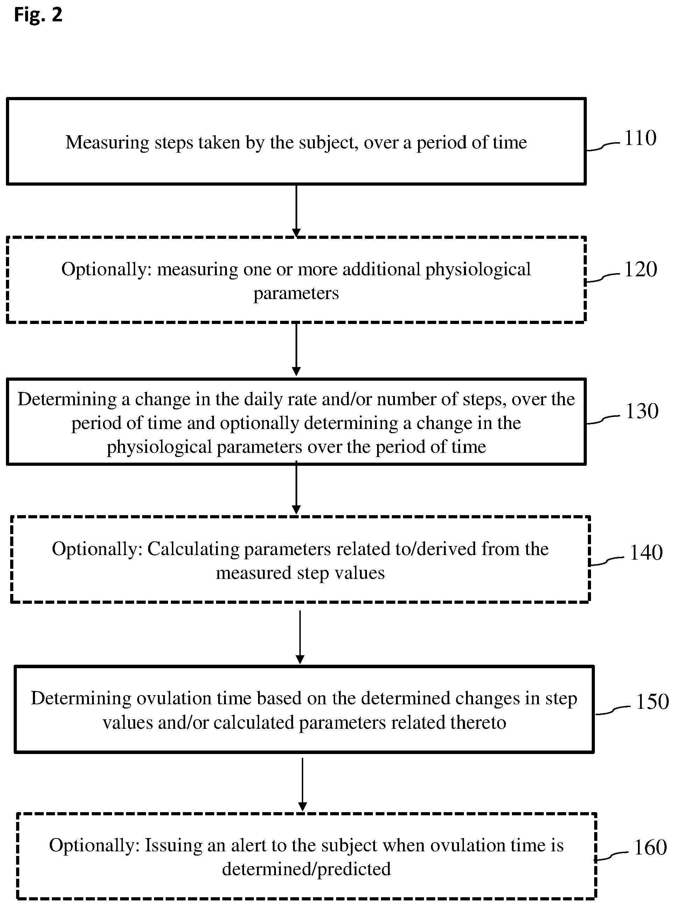

Reference is now made to **Fig. 2****,** which shows a block diagram of exemplary method for determining ovulation, based on changes in daily number and/or rate of steps, according to some embodiments. As shown in Fig. 2, at step **110,** the number of steps of the subject, is measured over a period of time, using a suitable sensor, harbored in a corresponding device, such as, for example, a wearable device. Next, at optional step **120,** one or more additional parameters may be measured (using suitable sensors), including, for example, but not limited to: heart rate, stress, sleep pattern, length of a night sleep, quality of a night sleep, sleep stages, ECG, oxygen saturation, and the like. Next, at step **130,** a change in the measured steps, including, for example, daily rate and/or number of steps over the period of time and optionally a change in the physiological parameters over the period of time is determined/calculated. In some embodiments, the determination of change may include any suitable calculation and statistical methods, including, for example, smoothing, averaging, normalizing, and the like. In some embodiments, the change is determined over the entire time period. In some embodiments, the change is a change over and between portions of the time period. In some embodiments, the change may be determined over and between the follicular stage, ovulation stage and/or post ovulation stage. At optional step **140,** the calculations may include calculating or determining various parameters related or derived from the measured steps values, including, for example, normalized values, averaged values, trends, and the like. In some embodiments, the trend is over the entire time period, or over and between parts of the time period. At step **150,** the ovulation time is determined/calculated/computed based on the determined changes obtained in any of the preceding steps of the method. In some embodiments, increase in the number of steps and/or rate of steps (including, for example, averaged and/or normalized values thereof) over the time period, or at least over portions of the time period is indicative of ovulation and can be used to determine or predict ovulation time/stage/period/occurrence. Optionally, at step 160, the method may further include a step of issuing an alert to the subject if/when ovulation time/occurrence is determined or expected/predicted. In some embodiments, the alert may include any type of alert, including, for example, tactile alert, visual alert, audible alert, text message, email and the like, or any combination thereof.

According to some embodiments, there is provided a method for determining or predicting ovulation time in human subject, the method includes:
measuring daily rate and/or number of steps of the subject, over a period of time; and
determining a change in a pattern of the daily rate and/or number of steps, over the period of time;
wherein the change is indicative of ovulation.

According to some embodiments, the method may further include measuring one or more physiological parameters of the subject and determining a change in the physiological parameters over the period of time.

According to some embodiments, the physiological parameters may be selected from: sleep pattern (including, for example, length of a night sleep, quality of a night sleep, sleep stages), heart rate, heart rate variability, respiration, ECG, skin temperature, body temperature, or any combination thereof.

According to some embodiments, the method may further include calculating a trend of the change in the number and/or rate of steps and/or a change in the measured physiological parameters.

According to some embodiments, an increase in the rate and/or number of steps and/or an increase in the trend is indicative of ovulation.

According to some embodiments, the time period is in the length of about 14-28 days.

According to some embodiments, one or more of the measurements may be performed continuously over the period of time. According to some embodiments, one or more of the measurements may be performed intermittently over the period of time. According to some embodiments, the daily number of steps and/or rate of steps may be measured continuously over the period of time. According to some embodiments, the change and/or trend in change may be determined over the entire time period and/or over and between portions of the time period.

According to some embodiments, at least one of the calculations may be performed using a processing unit. According to some embodiments, one or more of the measurements may be performed using a wearable device. According to some embodiments, the processing unit may be included or housed within the wearable device. According to some embodiments, the processing unit may be external to the wearable device. According to some embodiments, the data obtained by the wearable device may be processed by the processing unit, wherein the wearable device and the processing unit are configured to operate in conjunction. In some embodiments, the wearable device and the processing unit are functionally and/or physically associated. According to some embodiments, the method may further include a step of alerting the subject when ovulation period is expected or determined.

According to some embodiments, there is provided a wearable device for determining or predicting ovulation timing in a human subject, the device may include:
a sensor configured to measure a daily rate and/or number of steps of the subject wearing the device, over a period of time; and
a processing unit configured to determine a change in the daily rate and/or number of steps, over the period of time; wherein the change is indicative of ovulation.

According to some embodiments, the device may further include one or more sensors configured to measure one or more physiological parameters of the subject.

According to some embodiments, the physiological parameters measured or determined by the wearable device may include: sleep pattern (including, for example, length of a night sleep, quality of a night sleep, sleep stages), respiration, heart rate, ECG, temperature, or any combination thereof. Each possibility is a separate embodiment.

In some embodiments, the device may include one or more sensors, including, for example, accelerometer, gyroscope, temperature sensor, stress sensor, SpO2 sensor, heart rate sensor, respiration sensor, and the like, or any combination thereof.

According to some embodiments, the processing unit of the wearable device may be further configured to determine a change in the one or more physiological parameters.

According to some embodiments, the processing unit of the wearable device may be further configured to calculate a trend of the change in the number and/or rate of steps and/or a change in the measured physiological parameters.

According to some embodiments, an increase in the rate and/or number of steps and/or an increase in the trend, as determined or calculated by the processing unit of the wearable device, is indicative of ovulation.

According to some embodiments, the time period may be in the length of about 2-35 days. According to some embodiments, one or more of the measurements and/or the calculations may be performed continuously or essentially continuously over the period of time. According to some embodiments, one or more of the measurements and/or calculations may be performed intermittently over the period of time.

According to some embodiments, the daily number of steps and/or rate of steps may be measured continuously or essentially continuously over the period of time.

According to some embodiments, the wearable device may further include a feedback unit configured to issue indication regarding ovulation.

According to some embodiments, the indication may be issued as a visual indication (for example, text message), tactile indication (for example, vibration) and/or audible indication.

According to some embodiments, terms such as "processing", "computing", "calculating", "determining", "estimating", "assessing", "gauging" or the like, may refer to the action and/or processes of a computer or computing system, or similar electronic computing device, that manipulate and/or transform data, represented as physical (e.g. electronic) quantities within the computing system's registers and/or memories, into other data similarly represented as physical quantities within the computing system's memories, registers or other such information storage, transmission or display devices. Embodiments of the present disclosure may include apparatuses for performing the operations herein. The apparatuses may be specially constructed for the desired purposes or may include a general-purpose computer(s) selectively activated or reconfigured by a computer program stored in the computer. Such a computer program may be stored in a computer readable storage medium, such as, but not limited to, any type of disk including floppy disks, optical disks, CD-ROMs, magnetic-optical disks, read-only memories (ROMs), random access memories (RAMs), electrically programmable read-only memories (EPROMs), electrically erasable and programmable read only memories (EEPROMs), magnetic or optical cards, or any other type of media suitable for storing electronic instructions, and capable of being coupled to a computer system bus. The processes and displays presented are not inherently related to any particular computer or other apparatus. Various general-purpose systems may be used with programs in accordance with the teachings herein, or it may prove convenient to construct a more specialized apparatus to perform the desired method(s). In addition, embodiments of the present disclosure are not described with reference to any particular programming language. It will be appreciated that a variety of programming languages may be used to implement the teachings of the present disclosure as described herein.

Aspects of the disclosure may be described in the general context of computer-executable instructions, such as program modules, being executed by a computer. Generally, program modules include routines, programs, objects, components, data structures, and so forth, which perform particular tasks or implement particular abstract data types. Disclosed embodiments may also be practiced in distributed computing environments where tasks are performed by remote processing devices that are linked through a communications network. In a distributed computing environment, program modules may be located in both local and remote computer storage media including memory storage devices.

In the description and claims of the application, the words "include" and "have", and forms thereof, are not limited to members in a list with which the words may be associated.

As used herein, the term "about" may be used to specify a value of a quantity or parameter (e.g. the length of an element) to within a continuous range of values in the neighborhood of (and including) a given (stated) value. According to some embodiments, "about" may specify the value of a parameter to be between 80 % and 120 % of the given value.

Unless otherwise defined, all technical and scientific terms used herein have the same meaning as commonly understood by one of ordinary skill in the art to which this disclosure pertains. In case of conflict, the patent specification, including definitions, governs. As used herein, the indefinite articles "a" and "an" mean "at least one" or "one or more" unless the context clearly dictates otherwise.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable subcombination or as suitable in any other described embodiment of the disclosure. No feature described in the context of an embodiment is to be considered an essential feature of that embodiment, unless explicitly specified as such.

Although steps of methods according to some embodiments may be described in a specific sequence, methods of the disclosure may include some or all of the described steps carried out in a different order. A method of the disclosure may include a few of the steps described or all of the steps described. No particular step in a disclosed method is to be considered an essential step of that method, unless explicitly specified as such.

Although the disclosure is described in conjunction with specific embodiments thereof, it is evident that numerous alternatives, modifications and variations that are apparent to those skilled in the art may exist. Accordingly, the disclosure embraces all such alternatives, modifications and variations that fall within the scope of the appended claims. It is to be understood that the disclosure is not necessarily limited in its application to the details of construction and the arrangement of the components and/or methods set forth herein. Other embodiments may be practiced, and an embodiment may be carried out in various ways.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention.

### EXAMPLES

### Example 1 - measuring activity fertile female subject during menstrual cycle.

The aim of the prospective controlled study is to determine changes in activity of fertile human female subjects during the menstrual cycle, in correlation with the follicular stage and/or ovulation.

In this study, the measured activity included, inter alia, number of steps taken by fertile human female subjects during the menstrual cycle, in correlation with the follicular stage and/or ovulation, and various parameters related thereto are calculated, including, inter alia, average number of steps, daily number of steps, rate of steps, daily rate of steps based on measurements over an entire day or over interim time periods within the day ("intraday").

The study includes a total of 35 participants, which are under 40 years of old, having a regular period and optionally undergo fertility treatments based on natural (non-induced) menstrual cycle.

Each of the participants wear a step monitoring wearable device (Polar M430) starting at the 2^{nd} day of the period consecutively until three days post ovulation (as determined by LH levels in blood tests).

Additional measurements are taken or determined, including, ECG, heart rate, sleep pattern (including, length of sleep, quality of sleep (as determined, for example, by movement during sleep)), stress, SpO2 and temperature.

In addition, as control, to determine ovulation time, standard blood tests are performed during different stages/time points along the menstrual cycle.

The measurements (number of steps, rate and one or more of the additional measurements) are performed at least during the follicular stage, during LH-surge stage and after ovulation. Further, changes between the obtained measurements are compared therebetween over the entire measurement period, over interim time periods, and/or between various stages of the menstrual cycle.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention as claimed. It is to be understood that further trials are being conducted to establish clinical effects.

## Claims

1. A non-invasive method for determining or predicting ovulation timing in a human subject, the method comprising:
measuring (110) activity of the subject over a period of time, said activity comprising steps taken by the subject; and
determining (130) a pattern in the steps of the subject over the period of time;
wherein the pattern is indicative of ovulation.

2. The method according to claim 1, wherein the measured steps comprise: total number of steps, daily number of steps, number of steps per time unit, average number of steps, rate of steps, average rate of steps, daily rate of steps, length of steps, average length of steps, normalized values thereof, or any combination thereof.

3. The method according to any one of claims 1-2, wherein the measured activity of the subject further comprises body movement, amount of movement, intensity of movement, length of movement, rate of movement, velocity of movement, repetition of movement, posture changes, or any combination thereof.

4. The method according to any one of claim 1-2, wherein the activity of the subject is measured during waking hours of the subject.

5. The method according to any one of claims 1-3, wherein the pattern comprises one or more changes in values of the measured activity during one or more interim periods of the period of time, and/or one or more changes in parameters derived from the values of the measured activity during one or more interim periods of the period of time.

6. The method according to claim 4, wherein parameters derived from the values of the measured activity comprises normalized values of said parameters, intraday values of the parameters, trends of said values, or any combination thereof.

7. The method according to any one of claims 1-6, wherein an increase in daily number of steps, daily rate of steps, average number of daily steps, average number of steps and/or rate of steps, and/or in parameters derived therefrom, in an interim time period, is indicative of ovulation time.

8. The method according to any one of claims 1-7, further comprising measuring (120) one or more physiological parameters of the subject and determining a pattern in the physiological parameters over the period of time;
wherein the pattern of the one or more physiological parameters comprises one or more changes in the measured physiological parameters during one or more interim periods of the period of time, and/or one or more changes in parameters derived from the changes in the measured physiological parameters during one or more interim periods of the period of time

9. The method according to any one of claims 1-8, wherein the time period is in the length of about 2-45 days.

10. The method according to any one of claims 1-9, wherein at least one of the calculations is performed using a processing unit; and/or wherein one or more of the measurements are performed using a wearable device; and/or
wherein the method further comprises alerting the subject when ovulation time is occurring or is expected.

11. A wearable device for determining or predicting ovulation time in a human subject, the device comprising:
a sensor configured to measure activity of the subject, over a period of time, said activity comprising steps taken by the subject; and
a processing unit configured to determine a pattern of the measured steps, over the period of time;
wherein the pattern is indicative of ovulation.

12. The wearable device according to claim 11, wherein the measured activity of the subject further comprises body movement, amount of movement, intensity of movement, length of movement, rate of movement, velocity of movement, repetition of movement, posture changes, or any combination thereof.

13. The wearable device according to any one of claims 11-12, wherein the pattern determined by the processing unit comprises one or more changes in values of the measured activity during one or more interim periods of the period of time, and/or one or more changes in parameters derived from the values of the measured activity during one or more interim periods of the period of time; and/or wherein parameters derived from the values of the measured activity comprises normalized values of said parameters, intraday values of the parameters, trends of said measured values, or any combinations thereof.

14. The device according to claim 13, wherein an increase in daily number of steps, daily rate of steps, average number of daily steps, average number of steps and/or rate of steps, and/or in parameters derived therefrom, in an interim time period, as determined by the processing unit, are indicative of ovulation time.

15. The device according to any one of claims 11-14, further comprising one or more sensors for measuring one or more physiological parameters of the subject, said sensors are selected from: stress sensor, accelerometer, gyroscope, heart rate sensor, temperature sensor, SpO2 sensor, respiration sensor, or any combination thereof; and/or
wherein the device further comprises an alert unit configured to issue indication regarding ovulation occurrence.

## Patentansprüche

1. Nicht-invasives Verfahren zur Bestimmung oder Vorhersage des Zeitpunktes des Eisprungs bei einer menschlichen Patientin, wobei das Verfahren umfasst:
Messen (110) der Aktivität der Patientin über einen Zeitraum, wobei die Aktivität Schritte umfasst, die von der Patientin gemacht werden; und
Bestimmen (130) eines Musters in den Schritten der Patientin über den Zeitraum;
wobei das Muster einen Hinweis auf den Eisprung gibt.

2. Verfahren nach Anspruch 1, wobei die gemessenen Schritte umfassen: Gesamtzahl der Schritte, tägliche Anzahl der Schritte, Anzahl der Schritte pro Zeiteinheit, durchschnittliche Anzahl der Schritte, Schrittfrequenz, durchschnittliche Schrittfrequenz, tägliche Schrittfrequenz, Schrittlänge, durchschnittliche Schrittlänge, normalisierte Werte davon oder eine beliebige Kombination davon.

3. Verfahren nach einem der Ansprüche 1-2, wobei die gemessene Aktivität der Patientin ferner Körperbewegungen, Bewegungsumfang, Bewegungsintensität, Bewegungsdauer, Bewegungsfrequenz, Bewegungsgeschwindigkeit, Bewegungswiederholung, Haltungsänderungen oder eine beliebige Kombination davon umfasst.

4. Verfahren nach einem der Ansprüche 1-2, wobei die Aktivität der Patientin während der Wachzeiten der Patientin gemessen wird.

5. Verfahren nach einem der Ansprüche 1-3, wobei das Muster eine oder mehrere Änderungen der Werte der gemessenen Aktivität während eines oder mehrerer Interimszeiträume des Zeitraums und/oder eine oder mehrere Änderungen der Parameter, die aus den Werten der gemessenen Aktivität während eines oder mehrerer Interimszeiträume des Zeitraums abgeleitet werden, umfasst.

6. Verfahren nach Anspruch 4, wobei die aus den Werten der gemessenen Aktivität abgeleiteten Parameter normalisierte Werte der Parameter, tageszeitliche Werte der Parameter, Trends der Werte oder eine beliebige Kombination davon umfassen.

7. Verfahren nach einem der Ansprüche 1-6, wobei eine Zunahme der täglichen Schrittzahl, der täglichen Schrittfrequenz, der durchschnittlichen täglichen Schrittzahl, der durchschnittlichen Schrittfrequenz und/oder der daraus abgeleiteten Parameter in einem Interimszeitraum einen Hinweis auf den Zeitpunkt des Eisprungs gibt.

8. Verfahren nach einem der Ansprüche 1-7, ferner umfassend das Messen (120) eines oder mehrerer physiologischer Parameter der Patientin und das Bestimmen eines Musters in den physiologischen Parametern über den Zeitraum;
wobei das Muster der ein oder mehreren physiologischen Parameter eine oder mehrere Änderungen der gemessenen physiologischen Parameter während eines oder mehrerer Interimszeiträume des Zeitraums und/oder eine oder mehrere Änderungen von Parametern, die aus den Änderungen der gemessenen physiologischen Parameter während eines oder mehrerer Interimszeiträume des Zeitraums abgeleitet sind, umfasst.

9. Verfahren nach einem der Ansprüche 1-8, wobei der Zeitraum eine Länge von etwa 2-45 Tagen hat.

10. Verfahren nach einem der Ansprüche 1-9, wobei mindestens eine der Berechnungen unter Verwendung einer Verarbeitungseinheit durchgeführt wird; und/oder wobei eine oder mehrere der Messungen unter Verwendung einer tragbaren Vorrichtung durchgeführt werden; und/oder
wobei das Verfahren ferner umfasst, die Patientin zu benachrichtigen, wann der Zeitpunkt des Eisprungs eintritt oder erwartet wird.

11. Tragbare Vorrichtung zur Bestimmung oder Vorhersage des Zeitpunktes des Eisprungs bei einer menschlichen Patientin, wobei die Vorrichtung umfasst:
einen Sensor, der ausgelegt ist zum Messen der Aktivität der Patientin über einen Zeitraum, wobei die Aktivität Schritte umfasst, die von der Patientin gemacht werden; und
eine Verarbeitungseinheit, die dafür ausgelegt ist, ein Muster der gemessenen Schritte über den Zeitraum zu bestimmen;
wobei das Muster einen Hinweis auf den Eisprung gibt.

12. Tragbare Vorrichtung nach Anspruch 11, wobei die gemessene Aktivität der Patientin ferner Körperbewegungen, Bewegungsumfang, Bewegungsintensität, Bewegungsdauer, Bewegungsfrequenz, Bewegungsgeschwindigkeit, Bewegungswiederholung, Haltungsänderungen oder eine beliebige Kombination davon umfasst.

13. Tragbare Vorrichtung nach einem der Ansprüche 11-12, wobei das von der Verarbeitungseinheit bestimmte Muster eine oder mehrere Änderungen der Werte der gemessenen Aktivität während eines oder mehrerer Interimszeiträume des Zeitraums und/oder eine oder mehrere Änderungen der Parameter, die aus den Werten der gemessenen Aktivität während eines oder mehrerer Interimszeiträume des Zeitraums abgeleitet werden, umfasst; und/oder wobei die aus den Werten der gemessenen Aktivität abgeleiteten Parameter normalisierte Werte der Parameter, tageszeitliche Werte der Parameter, Trends der gemessenen Werte oder beliebige Kombinationen davon umfassen.

14. Vorrichtung nach Anspruch 13, wobei eine Zunahme der täglichen Schrittzahl, der täglichen Schrittfrequenz, der durchschnittlichen täglichen Schrittzahl, der durchschnittlichen Schrittzahl und/oder Schrittfrequenz und/oder der daraus abgeleiteten Parameter in einem Interimszeitraum wie durch die Verarbeitungseinheit bestimmt einen Hinweis auf den Zeitpunkt des Eisprungs gibt.

15. Vorrichtung nach einem der Ansprüche 11-14, ferner einen oder mehrere Sensoren zum Messen eines oder mehrerer physiologischer Parameter der Patientin umfassend, wobei die Sensoren ausgewählt sind aus:
Stresssensor, Beschleunigungsmesser, Gyroskop, Herzfrequenzsensor, Temperatursensor, SpO2-Sensor, Atmungssensor oder einer beliebigen Kombination davon; und/oder
wobei die Vorrichtung ferner eine Alarmeinheit umfasst, die dafür ausgelegt ist, eine Anzeige bezüglich des Eintretens des Eisprungs auszugeben.

## Revendications

1. Procédé non-invasif pour déterminer ou prédire le moment d'ovulation chez un sujet humain, le procédé comprenant :
la mesure (110) de l'activité du sujet sur une période de temps, ladite activité comprenant des pas réalisés par le sujet ; et
la détermination (130) d'un motif dans les pas du sujet sur la période de temps ;
le motif étant indicatif de l'ovulation.

2. Procédé selon la revendication 1, les pas mesurés comprenant : le nombre total de pas, le nombre quotidien de pas, le nombre de pas par unité de temps, le nombre moyen de pas, le taux de pas, le taux de pas moyen, le taux de pas quotidien, la longueur des pas, la longueur moyenne des pas, les valeurs normalisées de ceux-ci, ou toute combinaison de ceux-ci.

3. Procédé selon l'une quelconque des revendications 1 et 2, l'activité mesurée du sujet comprenant en outre un mouvement corporel, une quantité de mouvement, une intensité de mouvement, une longueur de mouvement, un taux de mouvement, une vitesse de mouvement, une répétition du mouvement, des changements de posture ou toute combinaison de ceux-ci.

4. Procédé selon l'une quelconque des revendications 1 et 2, l'activité du sujet étant mesurée pendant des heures de veille du sujet.

5. Procédé selon l'une quelconque des revendications 1 à 3, le motif comprenant un ou plusieurs changements de valeurs de l'activité mesurée pendant une ou plusieurs périodes intermédiaires de la période de temps, et/ou un ou plusieurs changements de paramètres dérivés des valeurs de l'activité mesurée pendant une ou plusieurs périodes intermédiaires de la période de temps.

6. Procédé selon la revendication 4, les paramètres dérivés des valeurs de l'activité mesurée comprenant des valeurs normalisées desdits paramètres, des valeurs intrajournalières des paramètres, des tendances desdites valeurs, ou toute combinaison de celles-ci.

7. Procédé selon l'une quelconque des revendications 1 à 6, une augmentation du nombre quotidien de pas, du taux de pas quotidien, du nombre moyen de pas quotidiens, du nombre moyen de pas et/ou du taux de pas, et/ou de paramètres dérivés de ceux-ci, dans une période de temps intermédiaire, étant indicative du temps d'ovulation.

8. Procédé selon l'une quelconque des revendications 1 à 7, comprenant en outre la mesure (120) d'un ou plusieurs paramètres physiologiques du sujet et la détermination d'un motif dans les paramètres physiologiques sur la période de temps ;
le motif du ou des paramètres physiologiques comprenant une ou plusieurs modifications des paramètres physiologiques mesurés pendant une ou plusieurs périodes intermédiaires de la période de temps, et/ou un ou plusieurs changements de paramètres dérivés des modifications des paramètres physiologiques mesurés pendant une ou plusieurs périodes intermédiaires de la période de temps.

9. Procédé selon l'une quelconque des revendications 1 à 8, la période de temps étant d'une durée d'environ 2 à 45 jours.

10. Procédé selon l'une quelconque des revendications 1 à 9, au moins un des calculs étant effectué en utilisant une unité de traitement ; et/ou une ou plusieurs des mesures étant effectuées en utilisant un dispositif portable ; et/ou
le procédé comprenant en outre l'alerte du sujet lorsque le temps d'ovulation survient ou est attendu.

11. Dispositif portable pour déterminer ou prédire une durée d'ovulation chez un sujet humain, le dispositif comprenant :
un capteur configuré pour mesurer l'activité du sujet, sur une période de temps, ladite activité comprenant les pas réalisés par le sujet ; et
une unité de traitement configurée pour déterminer un motif des pas mesurés, sur la période de temps ;
le motif étant indicatif de l'ovulation.

12. Dispositif portable selon la revendication 11, l'activité mesurée du sujet comprenant en outre un mouvement corporel, une quantité de mouvement, une intensité de mouvement, une longueur de mouvement, un taux de mouvement, une vitesse de mouvement, une répétition du mouvement, des changements de posture ou toute combinaison de ceux-ci.

13. Dispositif portable selon l'une quelconque des revendications 11 à 12, le motif déterminé par l'unité de traitement comprenant un ou plusieurs changements de valeurs de l'activité mesurée pendant une ou plusieurs périodes intermédiaires de la période de temps, et/ou un ou plusieurs changements de paramètres dérivés des valeurs de l'activité mesurée pendant une ou plusieurs périodes intermédiaires de la période de temps ; et/ou des paramètres dérivés des valeurs de l'activité mesurée comprenant des valeurs normalisées desdits paramètres, des valeurs intrajournalières des paramètres, des évolutions desdites valeurs mesurées, ou toute combinaison de ceux-ci.

14. Dispositif selon la revendication 13, une augmentation du nombre quotidien de pas, du taux de pas quotidien, du nombre moyen de pas quotidiens, du nombre moyen de pas et/ou du taux de pas, et/ou de paramètres dérivés de ceux-ci, dans une période de temps intermédiaire, telle que déterminée par l'unité de traitement, étant indicative du temps d'ovulation.

15. Dispositif selon l'une quelconque des revendications 11 à 14, comprenant en outre un ou plusieurs capteurs pour mesurer un ou plusieurs paramètres physiologiques du sujet, lesdits capteurs étant sélectionnés parmi : un capteur de contrainte, un accéléromètre, un gyroscope, un capteur de fréquence cardiaque, un capteur de température, un capteur de SpO2, un capteur de respiration ou toute combinaison de ceux-ci ; et/ou
le dispositif comprenant en outre une unité d'alerte configurée pour émettre une indication concernant l'occurrence d'ovulation.
